# FASCICULE DE BREVET EUROPEEN

(11) **EP 2 892 358 B1**
(45) Date de publication et mention de la délivrance du brevet: **20.01.2016**
(21) Numéro de dépôt: 13750681.2
(22) Date de dépôt: 13.08.2013
(51) Int. Cl.: A23B 4/22, A23L 3/3571

(54) **COMPOSITION COMPRENANT AU MOINS TROIS SOUCHES DIFFERENTES DE L. SAKEI POUR LA CONSERVATION DE PRODUITS COMESTIBLES**
ZUSAMMENSETZUNG MIT MINDESTENS DREI VERSCHIEDENEN STÄMMEN VON L. SAKEI ZUR KONSERVIERUNG VON LEBENSMITTELN
COMPOSITION COMPRISING AT LEAST THREE DIFFERENT STRAINS OF L. SAKEI FOR PRESERVING EDIBLE PRODUCTS

(30) Priorité: 13.08.2012 FR 1257785
(43) Date de publication de la demande: 15.07.2015
(73) Titulaire: Institut National de la Recherche Agronomique (INRA), 75007 Paris (FR)
(72) Inventeur: CHAILLOU, Stéphane, F-78910 Orgerus (FR); CHAMPOMIER-VERGES, Marie-Christine, F-92330 Sceaux (FR); ZAGOREC, Monique, F-44300 Nantes (FR)
(74) Mandataire: Regimbeau
(86) Numéro de dépôt international: PCT/EP2013/066886
(87) Numéro de publication internationale: WO 2014/026972

(56) Documents cités:
- WO-A1-2011/023675
- FR-A1- 2 809 404
- VERMEIREN L ET AL: "Evaluation of meat born lactic acid bacteria as protective cultures for the biopreservation of cooked meat products", INTERNATIONAL JOURNAL OF FOOD MICROBIOLOGY, ELSEVIER SCIENCE PUBLISHERS, AMSTERDAM, NL, vol. 96, no. 2, 1 novembre 2004 (2004-11-01), pages 149-164, XP004567738, ISSN: 0168-1605, DOI: 10.1016/J.IJFOODMICRO.2004.03.016 cité dans la demande

## Description

La présente invention se rapporte à une composition comprenant au moins trois souches différentes de bactéries *Lactobacillus sakei* (*L. sakei*), et à son utilisation pour la conservation de produits comestibles, de préférence de produits carnés, en particulier la viande de boeuf et encore plus particulièrement du carpaccio de boeuf.

Les produits camés sont des aliments qui, sans traitement de conservation, se dégradent rapidement, à une vitesse qui dépend de divers facteurs : acidité du produit, taux d'humidité ambiant, atmosphère modifiée, type et charge de bactéries, température... De nombreux traitements de conservation des aliments, et en particulier des produits carnés, sont connus (fermentation, fumaison, salage, séchage, addition de conservateurs, conditionnement sous vide...). Cependant, l'usage de conservateurs est règlementé. De plus, la plupart de ces traitements entraîne une transformation du produit frais (goût, couleur, texture). Il est également connu d'utiliser des cultures protectrices à des fins de bio-conservation, l'effet recherché étant alors d'assurer la qualité microbiologique sans modification organoleptique du produit, de manière à lui préserver son caractère frais. Ceci implique donc l'utilisation de bactéries, inoffensives pour la santé de l'homme, qui vont empêcher le développement d'autres espèces bactériennes indésirables, sans modifier la matrice alimentaire. Si la recherche pour la mise au point de bonnes cultures protectrices se développe depuis plusieurs années, la difficulté majeure réside dans le fait que la compétition entre les différentes espèces bactériennes sur des substrats alimentaires complexes résulte d'un ensemble de mécanismes encore mal compris (Devlieghere et al., 2004). Quelques exemples existent (en France : ferment lactique LLO pour la conservation des crevettes ; aux Etats Unis : BovamineMeat™ pour la conservation de la viande ; en Europe Holdbac™ et SafePro Range™ commercialisés par Danisco et Ch Hansen pour la conservation de produits carnés). Cependant, les produits existant ne ciblent souvent qu'une espèce bactérienne *(Listeria monocytogenes)* et ne concernent pas le carpaccio de boeuf.

Plus particulièrement, en ce qui concerne les produits carnés, la viande destinée à être consommée crue ou peu cuite (steaks tartare ou haché, carpaccio par exemple) ou les plats cuisinés, sont ceux qui peuvent présenter des problèmes importants de contamination avec une incidence sur la santé du consommateur. En effet, dans le cas des produits consommés crus ou peu cuits, les bactéries pathogènes ne sont pas détruites par la cuisson et dans le cas des produits cuisinés, rendus stériles par leur cuisson préalable, une contamination ultérieure peut être dangereuse car en absence d'espèce compétitrice, les bactéries nouvellement contaminantes peuvent se développer jusqu'à un degré élevé. C'est donc essentiellement sur ces types de produits que la recherche sur les cultures protectrices des produits camés s'est développée (Bredholt et al., 2001 ; Vermeiren et al., 2004 ; Vold et al., 2000). Il a été observé que la flore endogène de la viande, et en particulier *L. sakei*, contribuait à empêcher le développement de bactéries pathogènes telles que *Escherichia coli* O157:H7 dans la viande hachée. Des essais réalisés à l'aide de souches productrices de bactériocines anti-Listéria ont montré qu'elles limitaient le développement des Listérias sur des produits camés cuits. Cependant, l'efficacité des bactériocines dans les matrices alimentaires reste peu maîtrisée et l'éventail des cibles est limité à quelques espèces bactériennes.

Il existe une très grande diversité de souches de *L. sakei* dont la taille du génome varie de 1,8 Mb à 2,3 Mb. Cette large diversité se regroupe en une dizaine de classes génétiques au sein de l'espèce. Ainsi, les Inventeurs ont identifié différents marqueurs génétiques permettant de caractériser les souches de *L. sakei* (demandes internationales WO 2009/106491 et WO 2011/023675).

Malgré la connaissance de cette diversité, il est actuellement impossible de savoir s'il existe un assemblage 'idéal' de souches qui pourrait lutter le plus efficacement contre l'altération d'une ou de plusieurs espèces altérantes dans un produit carné, en particulier de type carpaccio de boeuf.

De manière surprenante, les Inventeurs ont mis au point une composition spécifique comprenant 3 classes de *L. sakei,* bien identifiée et traçable dans le produit, qui permet de lutter contre plusieurs espèces bactériennes d'altération des aliments, en particulier des produits carnés, plus particulièrement les produits camés crus ou peu cuits et tout particulièrement du carpaccio de boeuf et du steack haché de boeuf Ces différentes classes de *L. sakei* sont identifiables par des marqueurs spécifiques.

Ainsi, la présente invention se rapporte à une composition comprenant au moins trois souches différentes de bactéries *L. sakei,* dans laquelle :
- ladite première souche de bactéries *L. sakei* comprend dans son génome la séquence suivante :
   - SEQ ID NO: 35 ou une séquence présentant au moins 95% d'identité avec celle-ci,
- ladite deuxième souche de bactéries *L. sakei* comprend dans son génome la séquence suivante :
   - SEQ ID NO: 21 ou une séquence présentant au moins 95% d'identité avec celle-ci,
- ladite troisième souche de bactéries *L. sakei* comprend dans son génome la séquence suivante :
   - SEQ ID NO: 26 ou une séquence présentant au moins 95% d'identité avec celle-ci.

De préférence, ladite première souche de bactéries *L. sakei* comprend dans son génome les séquences suivantes :
- SEQ ID NO: 42 et
- SEQ ID NO: 35 ou une séquence présentant au moins 95% d'identité avec celle-ci,
de préférence ladite première souche de bactéries *L. sakei* comprend dans son génome les séquences suivantes :
- SEQ ID NO: 2 ou une séquence présentant au moins 95% d'identité avec celle-ci,
- SEQ ID NO: 34 ou une séquence présentant au moins 95% d'identité avec celle-ci,
- SEQ ID NO: 42, et
- SEQ ID NO: 35 ou une séquence présentant au moins 95% d'identité avec celle-ci;
de préférence ladite première souche de bactéries *L. sakei* comprend dans son génome les séquences suivantes :
- SEQ ID NO: 2 ou une séquence présentant au moins 95% d'identité avec celle-ci,
- SEQ ID NO: 4 ou une séquence présentant au moins 95% d'identité avec celle-ci,
- SEQ ID NO: 6 ou une séquence présentant au moins 95% d'identité avec celle-ci,
- SEQ ID NO: 7 ou une séquence présentant au moins 95% d'identité avec celle-ci,
- SEQ ID NO: 8 ou une séquence présentant au moins 95% d'identité avec celle-ci,
- SEQ ID NO: 9 ou une séquence présentant au moins 95% d'identité avec celle-ci,
- SEQ ID NO: 10 ou une séquence présentant au moins 95% d'identité avec celle-ci,
- SEQ ID NO: 13 ou une séquence présentant au moins 95% d'identité avec celle-ci,
- SEQ ID NO: 15 ou une séquence présentant au moins 95% d'identité avec celle-ci,
- SEQ ID NO: 19 ou une séquence présentant au moins 95% d'identité avec celle-ci,
- SEQ ID NO: 32 ou une séquence présentant au moins 95% d'identité avec celle-ci,
- SEQ ID NO: 34 ou une séquence présentant au moins 95% d'identité avec celle-ci,
- SEQ ID NO: 42,
- SEQ ID NO: 35 ou une séquence présentant au moins 95% d'identité avec celle-ci,
- SEQ ID NO: 36 ou une séquence présentant au moins 95% d'identité avec celle-ci,
- SEQ ID NO: 38 ou une séquence présentant au moins 95% d'identité avec celle-ci, et
de manière particulièrement préférée, ladite souche est la souche déposée auprès de la CNCM (Collection Nationale de Cultures de Microorganismes ; Institut Pasteur, 28 rue du Docteur Roux, F-75724 Paris CEDEX 15) le 19 novembre 2010 sous le numéro CNCM 1-4396.

De préférence, ladite deuxième souche de bactéries *L. sakei* comprend dans son génome les séquences suivantes :
- SEQ ID NO: 21 ou une séquence présentant au moins 95% d'identité avec celle-ci, et
- SEQ ID NO: 40 ou une séquence présentant au moins 95% d'identité avec celle-ci;
de préférence, ladite deuxième souche de bactéries *L. sakei* comprend dans son génome les séquences suivantes :
- SEQ ID NO: 16 ou une séquence présentant au moins 95% d'identité avec celle-ci,
- SEQ ID NO: 17 ou une séquence présentant au moins 95% d'identité avec celle-ci,
- SEQ ID NO: 21 ou une séquence présentant au moins 95% d'identité avec celle-ci, et
- SEQ ID NO: 40 ou une séquence présentant au moins 95% d'identité avec celle-ci;
de préférence ladite deuxième souche de bactéries *L. sakei* comprend dans son génome les séquences suivantes :
- SEQ ID NO: 4 ou une séquence présentant au moins 95% d'identité avec celle-ci,
- SEQ ID NO: 8 ou une séquence présentant au moins 95% d'identité avec celle-ci,
- SEQ ID NO: 9 ou une séquence présentant au moins 95% d'identité avec celle-ci,
- SEQ ID NO: 11 ou une séquence présentant au moins 95% d'identité avec celle-ci,
- SEQ ID NO: 12 ou une séquence présentant au moins 95% d'identité avec celle-ci,
- SEQ ID NO: 16 ou une séquence présentant au moins 95% d'identité avec celle-ci,
- SEQ ID NO: 17 ou une séquence présentant au moins 95% d'identité avec celle-ci,
- SEQ ID NO: 20 ou une séquence présentant au moins 95% d'identité avec celle-ci,
- SEQ ID NO: 21 ou une séquence présentant au moins 95% d'identité avec celle-ci,
- SEQ ID NO: 36 ou une séquence présentant au moins 95% d'identité avec celle-ci,
- SEQ ID NO: 38 ou une séquence présentant au moins 95% d'identité avec celle-ci, et
- SEQ ID NO: 40 ou une séquence présentant au moins 95% d'identité avec celle-ci,
de préférence ladite souche comprend en outre une, de préférence, 2, 3, 4, 5, 6, 7, 8, 9, 10, 11, 12, 13, et encore préférentiellement 7, 8, 9, 10, 11, 12 des séquences choisies dans le groupe constitué par les séquences: SEQ ID NO: 5, SEQ ID NO: 10, SEQ ID NO: 14, SEQ ID NO: 15, SEQ ID NO: 18, SEQ ID NO: 19, SEQ ID NO: 24, SEQ ID NO: 29, SEQ ID NO: 31, SEQ ID NO: 32, SEQ ID NO: 33, SEQ ID NO: 37, SEQ ID NO: 41 et ou une séquence présentant au moins 95% d'identité avec celles-ci respectivement,
et de manière particulièrement préférée, ladite souche est la souche déposée auprès de la CNCM le 19 novembre 2010 sous le numéro CNCM 1-4393.

De préférence, ladite troisième souche de bactéries *L. sakei* comprend dans son génome les séquences suivantes :
- SEQ ID NO: 23 ou une séquence présentant au moins 95% d'identité avec celle-ci, et
- SEQ ID NO: 26 ou une séquence présentant au moins 95% d'identité avec celle-ci,
de préférence ladite troisième souche de bactéries *L. sakei* comprend dans son génome au moins les séquences suivantes :
- SEQ ID NO :22 ou une séquence présentant au moins 95% d'identité avec celle-ci,
- SEQ ID NO: 23 ou une séquence présentant au moins 95% d'identité avec celle-ci, et
- SEQ ID NO: 26 ou une séquence présentant au moins 95% d'identité avec celle-ci,
de préférence ladite troisième souche de bactéries *L. sakei* comprend dans son génome au moins les séquences suivantes :
- SEQ ID NO: 7 ou une séquence présentant au moins 95% d'identité avec celle-ci,
- SEQ ID NO: 13 ou une séquence présentant au moins 95% d'identité avec celle-ci,
- SEQ ID NO: 15 ou une séquence présentant au moins 95% d'identité avec celle-ci,
- SEQ ID NO: 20 ou une séquence présentant au moins 95% d'identité avec celle-ci,
- SEQ ID NO: 22 ou une séquence présentant au moins 95% d'identité avec celle-ci,
- SEQ ID NO: 23 ou une séquence présentant au moins 95% d'identité avec celle-ci, SEQ ID NO: 26 ou une séquence présentant au moins 95% d'identité avec celle-ci,
et de préférence ladite souche comprend en outre une, de préférence, 2, 3, 4, 5, 6, 7, 8, 9, 10, 11, 12, 13, 14 ou 15 et encore préférentiellement 5, 6, 7, 8, 9 des séquences choisies dans le groupe constitué par les séquences: SEQ ID NO: 3, SEQ ID NO: 5, SEQ ID NO: 9, SEQ ID NO: 12, SEQ ID NO: 14, SEQ ID NO: 25, SEQ ID NO: 27, SEQ ID NO: 28, SEQ ID NO: 29, SEQ ID NO: 30, SEQ ID NO: 31, SEQ ID NO: 36, SEQ ID NO: 37, SEQ ID NO: 38, et SEQ ID NO: 41 ou une séquence présentant au moins 95% d'identité avec celles-ci respectivement,
et de manière particulièrement préférée, ladite souche est la souche déposée auprès de la CNCM le 19 novembre 2010 sous le numéro CNCM 1-4394.

Ainsi, de préférence, la composition selon la présente invention comprend:
- une première souche de bactéries *L. sakei* comprenant dans son génome les séquences suivantes :
   - SEQ ID NO: 42, et
   - SEQ ID NO: 35 ou une séquence présentant au moins 95% d'identité avec celle-ci,
- une deuxième souche de bactéries *L. sakei* comprenant dans son génome les séquences suivantes :
   - SEQ ID NO: 17 ou une séquence présentant au moins 95% d'identité avec celle-ci, et
   - SEQ ID NO: 40 ou une séquence présentant au moins 95% d'identité avec celle-ci;
- une troisième souche de bactéries *L. sakei* comprenant dans son génome les séquences suivantes :
   - SEQ ID NO: 23 ou une séquence présentant au moins 95% d'identité avec celle-ci, et
   - SEQ ID NO: 26 ou une séquence présentant au moins 95% d'identité avec celle-ci.

De manière particulièrement préférée, la composition selon la présente invention comprend :
- une première souche de bactéries *L. sakei* comprenant dans son génome les séquences suivantes :
   - SEQ ID NO: 2 ou une séquence présentant au moins 95% d'identité avec celle-ci,
   - SEQ ID NO: 34 ou une séquence présentant au moins 95% d'identité avec celle-ci,
   - SEQ ID NO: 42, et
   - SEQ ID NO: 35 ou une séquence présentant au moins 95% d'identité avec celle-ci,
- une deuxième souche de bactéries *L. sakei* comprenant dans son génome les séquences suivantes :
   - SEQ ID NO: 16 ou une séquence présentant au moins 95% d'identité avec celle-ci,
   - SEQ ID NO: 17 ou une séquence présentant au moins 95% d'identité avec celle-ci,
   - SEQ ID NO: 21 ou une séquence présentant au moins 95% d'identité avec celle-ci, et
   - SEQ ID NO: 40 ou une séquence présentant au moins 95% d'identité avec celle-ci; et
- une troisième souche de bactéries *L. sakei* comprenant dans son génome les séquences suivantes :
   - SEQ ID NO :22 ou une séquence présentant au moins 95% d'identité avec celle-ci,
   - SEQ ID NO: 23 ou une séquence présentant au moins 95% d'identité avec celle-ci, et
   - SEQ ID NO: 26 ou une séquence présentant au moins 95% d'identité avec celle-ci.

De manière particulièrement préférée, la composition selon la présente invention comprend :
- une première souche de bactéries *L. sakei* comprenant dans son génome les séquences suivantes :
   - SEQ ID NO: 2 ou une séquence présentant au moins 95% d'identité avec celle-ci,
   - SEQ ID NO: 4 ou une séquence présentant au moins 95% d'identité avec celle-ci,
   - SEQ ID NO: 6 ou une séquence présentant au moins 95% d'identité avec celle-ci,
   - SEQ ID NO: 7 ou une séquence présentant au moins 95% d'identité avec celle-ci,
   - SEQ ID NO: 8 ou une séquence présentant au moins 95% d'identité avec celle-ci,
   - SEQ ID NO: 9 ou une séquence présentant au moins 95% d'identité avec celle-ci,
   - SEQ ID NO: 10 ou une séquence présentant au moins 95% d'identité avec celle-ci,
   - SEQ ID NO: 13 ou une séquence présentant au moins 95% d'identité avec celle-ci,
   - SEQ ID NO: 15 ou une séquence présentant au moins 95% d'identité avec celle-ci,
   - SEQ ID NO: 19 ou une séquence présentant au moins 95% d'identité avec celle-ci,
   - SEQ ID NO: 32 ou une séquence présentant au moins 95% d'identité avec celle-ci,
   - SEQ ID NO: 34 ou une séquence présentant au moins 95% d'identité avec celle-ci,
   - SEQ ID NO: 42,
   - SEQ ID NO: 35 ou une séquence présentant au moins 95% d'identité avec celle-ci,
   - SEQ ID NO: 36 ou une séquence présentant au moins 95% d'identité avec celle-ci,
   - SEQ ID NO: 38 ou une séquence présentant au moins 95% d'identité avec celle-ci, et
- une deuxième souche de bactéries *L. sakei* comprenant dans son génome les séquences suivantes :
   - SEQ ID NO: 4 ou une séquence présentant au moins 95% d'identité avec celle-ci,
   - SEQ ID NO: 8 ou une séquence présentant au moins 95% d'identité avec celle-ci,
   - SEQ ID NO: 9 ou une séquence présentant au moins 95% d'identité avec celle-ci,
   - SEQ ID NO: 11 ou une séquence présentant au moins 95% d'identité avec celle-ci,
   - SEQ ID NO: 12 ou une séquence présentant au moins 95% d'identité avec celle-ci,
   - SEQ ID NO: 16 ou une séquence présentant au moins 95% d'identité avec celle-ci,
   - SEQ ID NO: 17 ou une séquence présentant au moins 95% d'identité avec celle-ci,
   - SEQ ID NO: 20 ou une séquence présentant au moins 95% d'identité avec celle-ci,
   - SEQ ID NO: 21 ou une séquence présentant au moins 95% d'identité avec celle-ci,
   - SEQ ID NO: 36 ou une séquence présentant au moins 95% d'identité avec celle-ci,
   - SEQ ID NO: 38 ou une séquence présentant au moins 95% d'identité avec celle-ci,
   - SEQ ID NO: 40 ou une séquence présentant au moins 95% d'identité avec celle-ci,
   de préférence ladite souche comprenant en outre une, de préférence, 2, 3, 4, 5, 6, 7, 8, 9, 10, 11, 12, 13, 14, 15, 16 et encore préférentiellement 7, 8, 9, 10, 11, 12 des séquences choisies dans le groupe constitué par les séquences: SEQ ID NO: 5, SEQ ID NO: 10, SEQ ID NO: 14, SEQ ID NO: 15, SEQ ID NO: 18, SEQ ID NO: 19, SEQ ID NO: 24, SEQ ID NO: 29, SEQ ID NO: 31, SEQ ID NO: 32, SEQ ID NO: 33, SEQ ID NO: 37, SEQ ID NO: 41 et ou une séquence présentant au moins 95% d'identité avec celles-ci respectivement, et
- une troisième souche de bactéries *L. sakei* comprenant dans son génome au moins les séquences suivantes :
   - SEQ ID NO :22 ou une séquence présentant au moins 95% d'identité avec celle-ci,
   - SEQ ID NO: 23 ou une séquence présentant au moins 95% d'identité avec celle-ci, et
   - SEQ ID NO: 26 ou une séquence présentant au moins 95% d'identité avec celle-ci,
   de préférence ladite troisième souche de bactéries *L. sakei* comprenant dans son génome au moins les séquences suivantes :
   - SEQ ID NO: 7 ou une séquence présentant au moins 95% d'identité avec celle-ci,
   - SEQ ID NO: 13 ou une séquence présentant au moins 95% d'identité avec celle-ci,
   - SEQ ID NO: 15 ou une séquence présentant au moins 95% d'identité avec celle-ci,
   - SEQ ID NO: 20 ou une séquence présentant au moins 95% d'identité avec celle-ci,
   - SEQ ID NO: 22 ou une séquence présentant au moins 95% d'identité avec celle-ci,
   - SEQ ID NO: 23 ou une séquence présentant au moins 95% d'identité avec celle-ci,
   - SEQ ID NO: 26 ou une séquence présentant au moins 95% d'identité avec celle-ci,
   et de préférence ladite souche comprend en outre une, de préférence, 2, 3, 4, 5, 6, 7, 8, 9, 10, 11, 12, 13, 14, 15, 16, 17 ou 18 et encore préférentiellement 5, 6, 7, 8, 9 des séquences choisies dans le groupe constitué par les séquences: SEQ ID NO: 3, SEQ ID NO: 5, SEQ ID NO: 9, SEQ ID NO: 12, SEQ ID NO: 14, SEQ ID NO: 25, SEQ ID NO: 27, SEQ ID NO: 28, SEQ ID NO: 29, SEQ ID NO: 30, SEQ ID NO: 31, SEQ ID NO: 36, SEQ ID NO: 37, SEQ ID NO: 38, et SEQ ID NO: 41 ou une séquence présentant au moins 95% d'identité avec celles-ci respectivement.

De préférence, la présente invention se rapporte à une composition telle que définie ci-dessus comprenant au moins une quatrième souche de bactéries *L. sakei,* différente des autres souches définies ci-dessus.

De préférence, ladite quatrième souche de bactéries *L. sakei* comprend dans son génome au moins la séquence SEQ ID NO : 39 ou une séquence présentant au moins 95% d'identité avec celle-ci.

Dans un mode de réalisation préféré, la quatrième souche comprend dans son génome au moins les séquences suivantes :
- SEQ ID NO: 4 ou une séquence présentant au moins 95% d'identité avec celle-ci,
- SEQ ID NO: 6 ou une séquence présentant au moins 95% d'identité avec celle-ci,
- SEQ ID NO: 7 ou une séquence présentant au moins 95% d'identité avec celle-ci,
- SEQ ID NO: 8 ou une séquence présentant au moins 95% d'identité avec celle-ci,
- SEQ ID NO: 9 ou une séquence présentant au moins 95% d'identité avec celle-ci,
- SEQ ID NO: 15 ou une séquence présentant au moins 95% d'identité avec celle-ci,
- SEQ ID NO: 31 ou une séquence présentant au moins 95% d'identité avec celle-ci,
- SEQ ID NO: 36 ou une séquence présentant au moins 95% d'identité avec celle-ci,
- SEQ ID NO: 39 ou une séquence présentant au moins 95% d'identité avec celle-ci,
et de préférence ladite souche comprend en outre une, de préférence, 1, 2, 3, 4, 5 et encore préférentiellement 1 ou 2 des séquences choisies dans le groupe constitué par les séquences: SEQ ID NO: 10, SEQ ID NO: 12, SEQ ID NO: 19, SEQ ID NO: 32 et SEQ ID NO: 38 ou une séquence présentant au moins 95% d'identité avec celles-ci respectivement, et de manière particulièrement préférée, ladite souche est la souche déposée auprès de la CNCM le 19 novembre 2010 sous le numéro CNCM 1-4395.

Le génome des 4 souches de *L. sakei* déposées auprès de la CNCM sous les numéros suivants: CNCM 1-4393, CNCM 1-4394, CNCM 1-4395 et CNCM 1-4396 a été séquencé par les inventeurs.

De préférence, ladite composition est une composition alimentaire. Au sens de la présente invention, on entend par « composition alimentaire », une composition susceptible d'être intégrée dans un produit alimentaire, c'est-à-dire une composition apte à la consommation. On entend par « apte à la consommation », un produit assimilable par individu, de préférence un humain, en toute innocuité, c'est-à-dire n'étant pas susceptible d'engendrer une pathologie à court terme.
De préférence, ladite composition est formulée de manière à garantir la viabilité desdites au moins trois souches, de préférence desdites au moins quatre souches.

De préférence, au sens de la présente invention, les séquences nucléotidiques utilisées comme marqueurs des souches de *L.sakei* selon la présente invention correspondent une séquence nucléotidique présentant au moins 95 %, 96 %, 97 %, 98 %, 99 % d'identité avec les séquences définies ci-avant.

Par séquence nucléotidique présentant au moins 95 % d'identité, de préférence au moins 96 %, 97 %, 98 %, 99 % d'identité avec une séquence, on entend désigner les polynucléotides présentant, par rapport au polynucléotide de référence, certaines modifications comme en particulier une délétion, une troncation, un allongement, une fusion chimérique et/ou une substitution, notamment ponctuelle. Il s'agit de préférence de séquences codant les mêmes séquences amino-acides que la séquence de référence, ceci lié à la dégénérescence du code génétique, ou de séquences complémentaires qui sont susceptibles de s'hybrider spécifiquement avec les séquences nucléotidiques de référence, de préférence dans des conditions de forte stringence.

Une hybridation dans des conditions de forte stringence signifie que les conditions de température et de force ionique sont choisies de telle manière à ce qu'elles permettent le maintien de l'hybridation entre deux fragments d'ADN complémentaires.

Les pourcentages d'identité auxquels il est fait référence dans le cadre de l'exposé de la présente invention sont déterminés sur la base d'un alignement global des séquences à comparer, c'est-à-dire sur un alignement des séquences prises dans leur intégralité, en utilisant par exemple l'algorithme de NEEDLEMAN et WUNSCH (J. Mol. Biol. 48, 443-453, 1970). Cette comparaison de séquences peut être effectuée par exemple à l'aide du logiciel needle en utilisant le paramètre "Gap open" égal à 10.0, le paramètre "Gap Extend" égal à 0.5, et une matrice « Blosum 62 ». Le logiciel needle est par exemple disponible sur le site internet ebi.ac.uk world wide, sous la dénomination « Align ».

De manière particulièrement préférée, la composition telle que définie précédemment comprend au moins une, de préférence deux, de préférence trois, et encore préférentiellement quatre souches de *L. sakei* choisies dans le groupe consistant en : les souches déposées auprès de la CNCM le 19 novembre 2010 sous les numéros suivants : CNCM 1-4393, CNCM 1-4394, CNCM 1-4395 et CNCM 1-4396.

Ainsi, de préférence, la composition telle que définie précédemment comprend au moins les 4 souches de *L. sakei* déposées auprès de la CNCM sous les numéros suivants : CNCM 1-4393, CNCM 1-4394, CNCM 1-4395 et CNCM 1-4396.

Dans un mode de réalisation particulier, la composition telle que définie précédemment ne comprend pas d'autres souches de *L. sakei* que les quatre souches telles que définies ci-dessus.

De préférence, la composition selon la présente invention peut comprendre d'autres agents conservateurs choisis dans le groupe comprenant : les marinades, tels que les marinades à base de vin rouge ou de soja, les huiles essentielles et les bactériocines.

De préférence, la composition selon la présente invention comprend les au moins quatre souches à des concentrations équivalentes.

De préférence, lesdites souches sont présentes à des concentrations permettant la réduction désirée des flores cibles dans le produit comestible. De préférence, chacune desdites souches est présente dans la composition à une concentration comprise entre 10³ et 10⁸ cfu/ml, de préférence à une concentration comprise entre 10⁴ et 10⁷ cfu/ml, de manière particulièrement préférée à une concentration comprise entre 10⁵ et 10⁶ cfu/ml. Un autre aspect de la présente invention se rapporte à une méthode de préparation de la composition selon la présente invention comprenant une étape de mise en culture d'au moins trois souches, de préférence au moins quatre souches de *L. sakei* telles que définies ci-avant dans une composition alimentaire.

De préférence, lesdites au moins trois souches, de préférence au moins quatre souches de *L. sakei* sont introduites à des concentrations équivalentes.

De préférence, les au moins trois souches, de préférence au moins quatre souches de *L. sakei* sont introduites à une concentration comprise entre 10³ et 10⁸ cfu/ml de composition alimentaire, de préférence à une concentration comprise entre 10⁴ et 10⁷ cfu/ml de composition alimentaire, de manière particulièrement préférée à une concentration comprise entre 10⁵ et 10⁶ cfu/ml de composition alimentaire.

Un autre aspect de la présente invention se rapporte à l'utilisation d'une composition telle que définie ci-avant pour la conservation d'un produit comestible, de préférence d'un produit carné, en particulier de la viande de boeuf et encore plus particulièrement du carpaccio de boeuf ou du boeuf haché et de manière particulièrement préférée du carpaccio de boeuf.

De préférence, le produit camé est cru ou peu cuit.

Le boeuf haché peut par exemple être destiné à être consommé en tant que tartare de boeuf.

La composition selon la présente invention permet une conservation du produit comestible grâce à une inhibition de la croissance de certains microoganismes susceptibles de générer une altération dudit produit comestible, notamment au cours du stockage du produit.

La mesure de l'altération peut être évaluée par la différence de la concentration desdits microorganismes entre le temps initial et le temps final de stockage, et peut alors être exprimée en ΔLog₁₀ CFU/g de produit comestible entre le temps final et le temps initial.

L'altération dudit produit comestible peut dans certains cas générer une réponse pathologique chez un individu.

A titre d'exemple de microorganismes susceptibles de générer une altération du produit comestible, on peut citer par exemple les *Enterobactéries du groupe Serratia (Serratia, Hafnia, Obesumbacterium, Rahnella),* les *Leuconostocs* du groupe *mesenteroides (mesenteroides-carnosum-gasicomitatum-gelidum),* les *Pseudomonas* choisies parmi les espèces *P. fragi, P. putida et P. fluorescens,* et quelques espèces plus spécifiquement telles que *Hafnia alvei, Brochothrix thermosphacta et Weisseila viridescens ...*

L'altération est un phénomène lié à la croissance des espèces altérantes et à la quantité de population atteinte par ces espèces au cours du stockage.

Au sens de la présente invention, on entend par « carpaccio » des tranches fines, de préférence comprise entre 0,1 mm et 1 mm d'épaisseur, d'un produit comestible cru.

Un autre aspect de la présente invention se rapporte à une méthode de conservation d'un produit comestible tel que défini précédemment comprenant une étape de mise en contact dudit produit comestible avec une composition telle que définie précédemment.

De préférence, ladite étape de mise en contact est réalisée avant l'étape de conditionnement dudit produit comestible.

De préférence, l'étape de mise en contact est réalisée par trempage dudit produit comestible dans une composition telle que définie précédemment.

De préférence, ledit produit comestible est trempée dans la composition telle que définie précédemment pendant 1 à 30 secondes, de préférence 2 à 10 secondes encore préférentiellement 4 à 6 secondes et de manière particulièrement préférée pendant 5 secondes.

Alternativement, l'étape de mise en contact est réalisée par pulvérisation de la composition selon la présente invention sur ledit produit comestible.

Au sens de la présente invention, on entend par « pulvérisation » la vaporisation de ladite composition sur ledit produit comestible.

Alternativement, l'étape de mise en contact est réalisée par mélange de la composition selon la présente invention, de préférence sous forme lyophilisée, avec ledit produit comestible.

De préférence, l'étape de mise en contact réalisée par mélange de la composition selon la présente invention, de préférence sous forme lyophilisée, avec ledit produit comestible est mise en oeuvre lorsque ledit produit comestible est du steak haché.

De préférence, l'étape de mise en contact est réalisée après l'étape de tranchage ou de hachage du produit comestible, de préférence de la viande.

De manière particulièrement préférée, l'étape de mise en contact est réalisée moins de 1 heure, de préférence, moins de 30 minutes, moins de 15 minutes, moins de 10 minutes, moins de 5 minutes et de préférence moins de 1 minute après l'étape de tranchage ou de hachage du produit comestible, de préférence de la viande.

De préférence, l'étape de mise en contact est suivie par une étape de mise sous vide ou de mise sous atmosphère protectrice (telle qu'une atmosphère comprenant de l'azote, du dioxyde de carbone, et de l'oxygène) dudit produit comestible.

De manière particulièrement préférée, l'étape de mise sous vide est réalisée moins de 1 heure, de préférence, moins de 30 minutes, moins de 15 minutes, moins de 10 minutes, moins de 5 minutes et de préférence moins de 1 minute après l'étape de mise en contact.

Enfin, la présente invention se rapporte à un produit comestible comprenant une composition telle que définie précédemment.

De préférence, ledit produit comestible est choisi parmi les produits carnés, en particulier d'un produit camé cru ou peu cuit.

De préférence ledit produit carné, en particulier ledit produit camé cru ou peu cuit est une viande de boeuf, plus particulièrement du carpaccio de boeuf ou du boeuf haché et de manière particulièrement préférée du carpaccio de boeuf.

Le boeuf haché peut exemple être destiné à être consommé en tant que tartare de boeuf.

De préférence chacune desdites souches est présente dans ledit produit comestible à une concentration comprise entre 10² et 10⁷ cfu/g de produit comestible, de préférence à une concentration comprise entre 10³ et 10⁶ cfu/g de produit comestible, de manière particulièrement préférée à une concentration comprise entre 10⁴ et 10⁵ cfu/g de produit comestible.

De préférence, ledit produit comestible comprend une concentration totale de souches de la composition comprise entre 10³ et 10⁹ cfu/g de produit comestible, de préférence entre 10³ et 10⁴ cfu/g et 10⁸ cfu/g de produit comestible après la mise en contact avec la composition selon la présente invention.

De préférence, ledit produit comestible comprend une concentration totale de souches de la composition comprise entre 10⁴ cfu/g et 10⁸ cfu/g de produit comestible, de préférence comprise entre 10⁵ cfu/g et 10⁷ cfu/g de produit comestible à la date limite de consommation.

### Description des figures

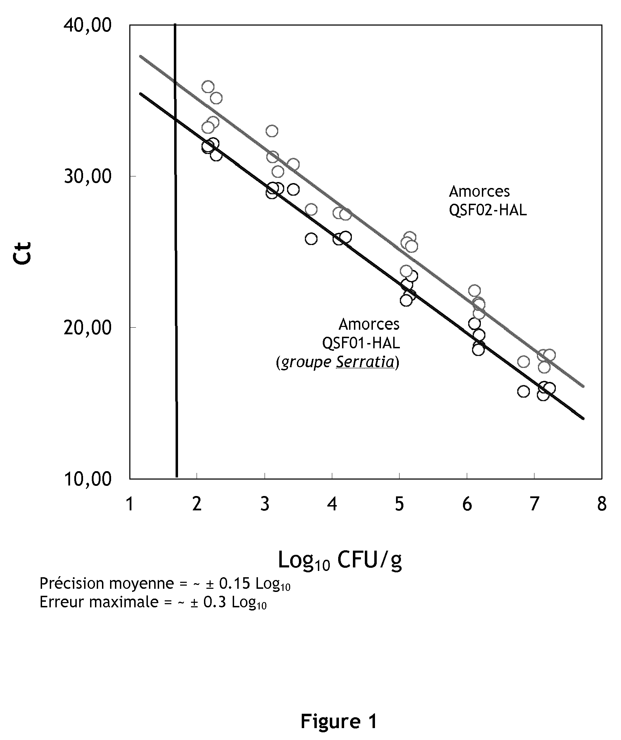

La figure 1 représente un exemple de correspondance Ct/CFU où l'on observe que les données de q-PCR sont linéaires et corrélées à la quantité de bactéries dans une gamme de 10² à 10⁷ CFU/g de carpaccio. La figure montre également un exemple où suivant l'amorce utilisée, on peut quantifier soit l'espèce *Hafnia alvei,* soit plus globalement le groupe bactérien (*Serratia*) auquel *H. alvei* est apparenté.
   Chaque rond correspond à une donnée expérimentale, les données donnent une droite. La précision de 0,1 Log10 et la marge d'erreur de 0,3 Log10 des données obtenues par q-PCR sont dans l'ensemble plus faibles que celles connues pour les comptages bactériens réalisés sur milieux de culture.

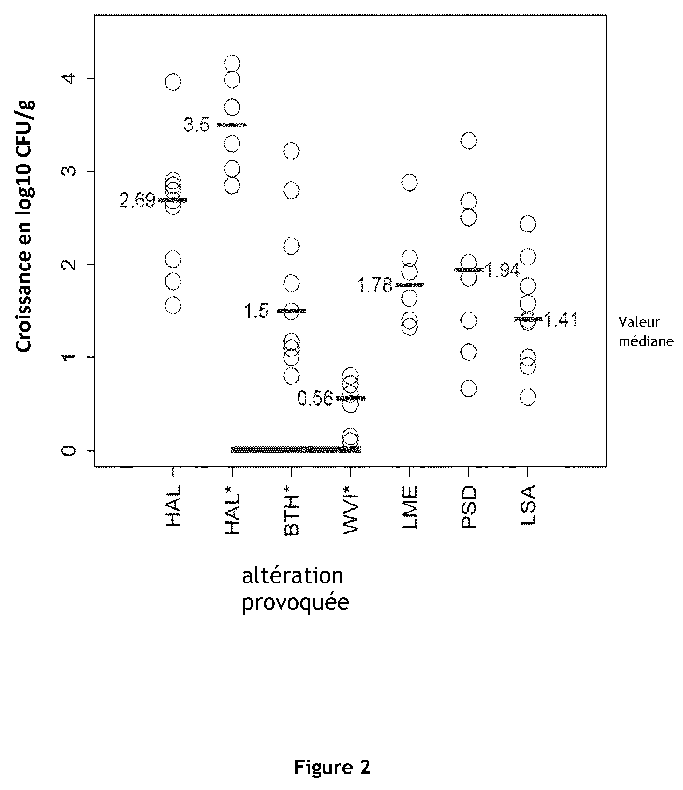

La figure 2 montre le résultat des quantifications des différentes espèces (genres/groupes). Chaque rond donne la croissance en ΔLog₁₀ (Log₁₀ à T₁₄ⱼₒᵤᵣₛ après stockage sous-vide et à 8°C, incluant un choc à 22°C pendant 24 heures au 7ème jour - Log₁₀ à T₀) des différentes espèces/groupes/genres. Chaque rond correspond à une expérience (soit un lot de carpaccio du commerce). (SER : groupe *Serratia*, HAL : espèce *Hafnia alvei,* BTH : espèce *Brochothrix thermosphacta*, WVI: espèce *Weisella viridescens*, LME: genre *Leuconostoc* (4 espèces), PSD: genre (3 espèces) *Pseudomonas*, LSA: espèce *Lactobacillus sakei*). On observe une grande variabilité d'un lot à l'autre: le groupe *Serratia* par exemple peut se développer, au cours des 14 jours, de environ 1.5 à environ 4 Log₁₀ par exemple avec une valeur médiane de 2,69 Log₁₀.

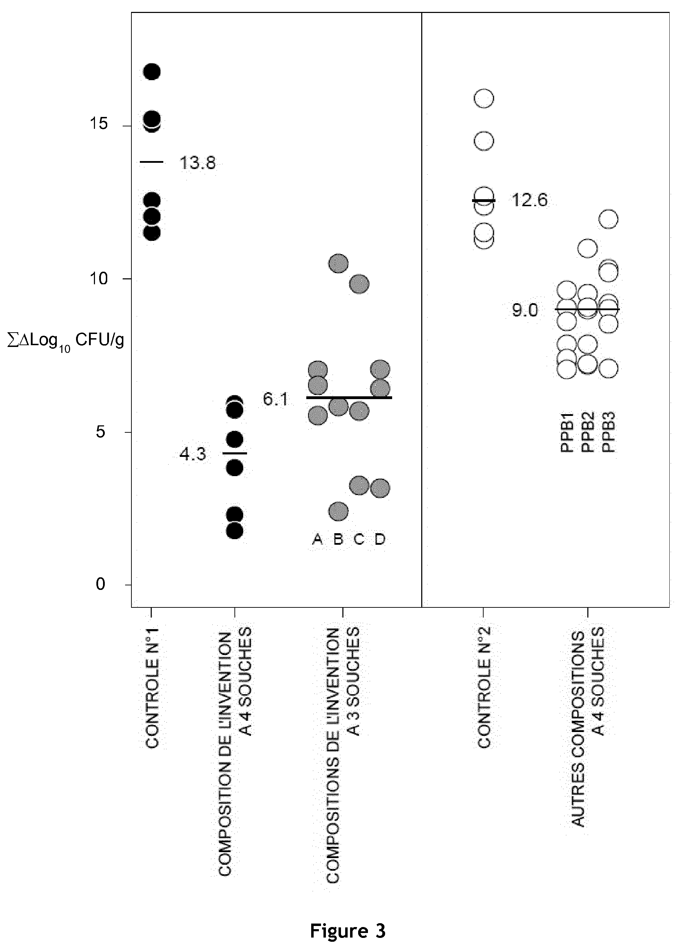

La figure 3 montre les effets des différents composants de la composition sur la croissance des espèces cibles. La mesure de ces effets se réalise par la comparaison des cumuls de croissance des différentes espèces cibles ou CGE (cumulative growth equivalent) (cinq mesures différentes cumulées).
   Cette valeur de CGE est schématisée sous forme de cercles de couleurs différentes selon le type de comparaison effectuée (l'échelle est exprimée en ∑ΔLog10 CFU/g de viande et est mesurée à T14 jours pour les différents échantillons (voir description conditions de stockage dans la légende figure 2). La valeur médiane obtenue à partir des différents lots (∑ΔLog10 CFU/g) est indiquée par un chiffre adjacent à un tiret horizontal. Les valeurs du contrôle N°1 (6 lots sans ajout de composition) peuvent être comparées avec celles obtenues avec les même lots traités avec la composition de l'invention à 4 souches (cercles noirs) et avec les compositions de l'invention à 3 souches (cercles gris, 3 lots seulement). Les codes des compositions de l'invention à 3 souches sont : A= souches CNCM 1-4395, CNCM 1-4393, CNCM I-4394; B = souches CNCM 1-4396, CNCM I-4393, CNCM I-4394; C = Souches CNCM 1-4396, CNCM 1-4395, CNCM 1-4393 et D = Souches CNCM 1-4396, CNCM 1-4395, CNCM 1-4394.
   Les valeurs du contrôle N°2 (6 autres lots sans ajout de composition) peuvent être comparées avec celles obtenues avec les même lots traités avec d'autres compositions à 4 souches utilisées lors du processus de criblage soit 3 séries (notées PPB1 à PPB3) de 7 compositions.
La figure 4 schématise la quantification à T14 jours des souches de la composition de l'invention par q-PCR sur l'ADN extrait des carpaccios à l'aide d'amorces spécifiques de cinq marqueurs. Le marqueur *katA* est spécifique de l'espèce *L. sakei* (présent chez toutes les souches c'est-à-dire celles de la composition mais aussi celles de la flore de *L. sakei* naturellement présente sur la viande). Les marqueurs SEQ ID NO :35, SEQ ID NO :39, SEQ ID NO :21 et SEQ ID NO :26 sont chacun spécifique d'une seule des 4 souches de la composition.

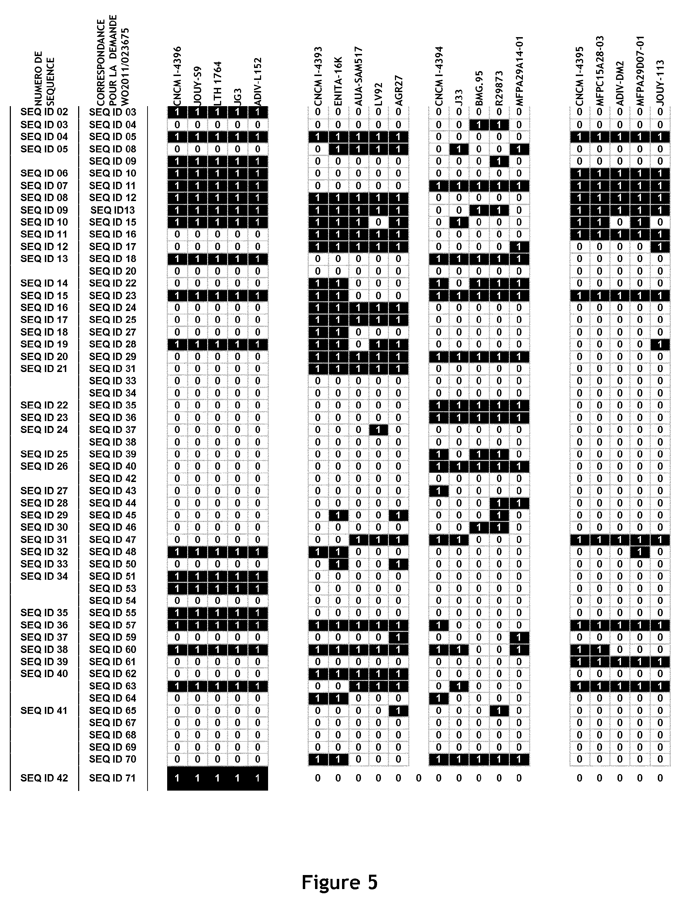

Les figures 5 à 7 se rapportent à la définition des classes de souches par rapport aux souches de la composition selon la présente invention et en fonction de l'empreinte moléculaire.
La figure 6 définit des classes de souches par rapport aux souches de la composition de l'invention et en fonction de l'empreinte moléculaire à 52 marqueurs de 4 autres souches proches.

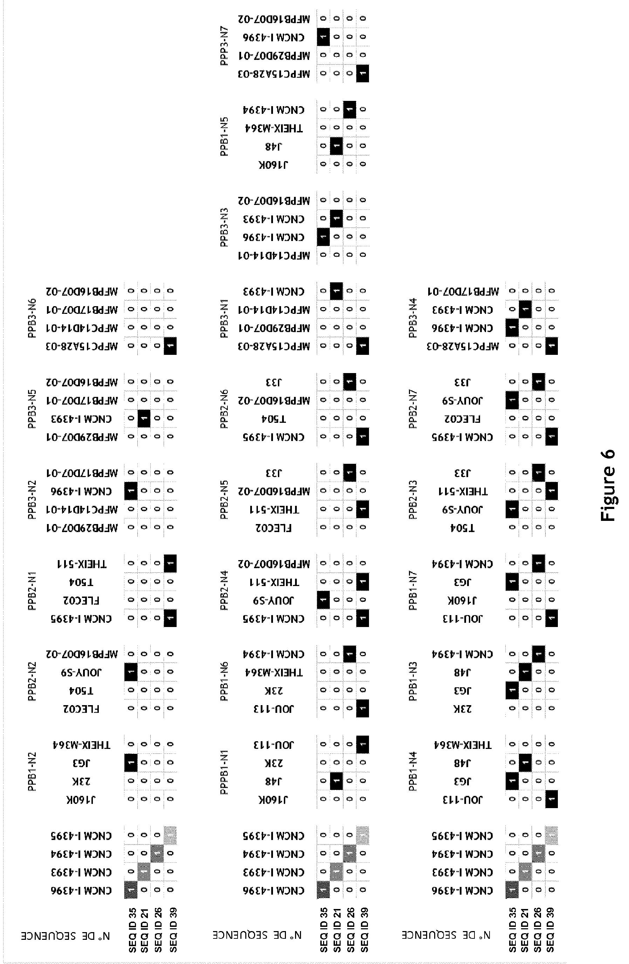

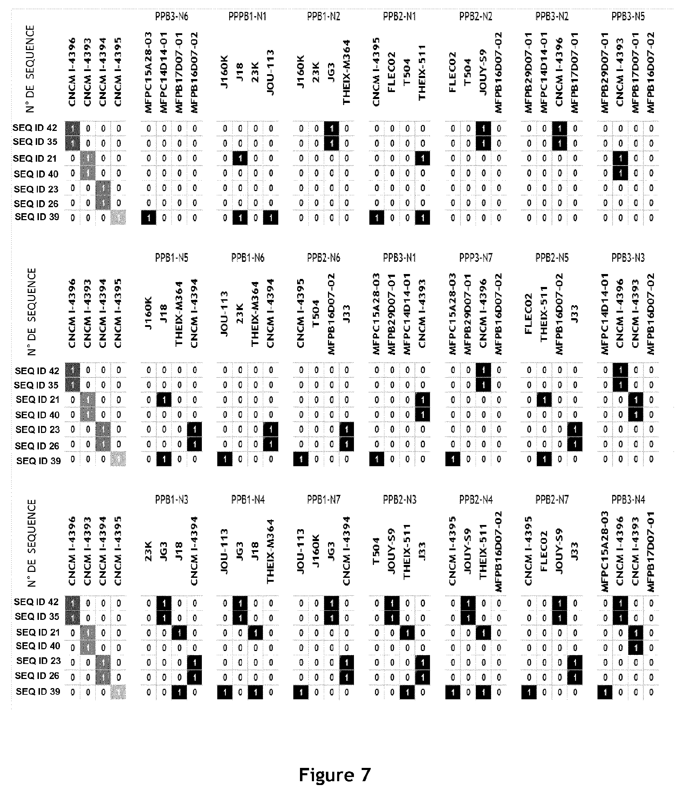

Les figures 6 et 7 montrent une comparaison des empreintes moléculaires entre la composition de l'invention à 4 souches et les 21 autres compositions à 4 souches avec 4 ou 7 marqueurs spécifiques.

### Exemples

### 1) Protocole d'altération forcée du Carpaccio de Boeuf

Les expériences ont été réalisées sur des carpaccios achetés dans le commerce, donc naturellement contaminés.

### a- quantification des bactéries altérantes naturellement présentes dans le carpaccio

Certains genres ou espèces de bactéries altérantes étaient systématiquement présents et facilement quantifiables. Ils ont donc été quantifiés sans nécessité d'ajout supplémentaire au carpaccio (*Enterobactéries du groupe Serratia (dont Hafnia alvei) (SER), Leuconostocs (mesenteroides-carnosum-gasicomitatum-gelidum)* et *Pseudomonas (fragi-putida-fluorescens)* (*PSD*)).

### b- quantification des bactéries altérantes après ensemencement volontaire du carpaccio

Au contraire, d'autres genres ou espèces de bactéries altérantes pouvaient être présents mais plus difficilement quantifiables, ou se développant peu, ou à des taux très variables. Ceux-ci ont été quantifiés à la fois comme contaminants naturels et après ensemencement volontaire (*Hafnia alvei,* souche CIP57.31 *(HAL) Brochothrix thermosphacta*, souche INRA 160*7 *(BTH), Weisseila viridescens*, souche CIP102810 *(WVI)).*

Un protocole d'inoculation volontaire a donc dû être mis au point où une suspension bactérienne de charge connue (en CFU/ml de suspension) était utilisée pour ensemencer les carpaccios par trempage/égouttage puis la charge des carpaccios était mesurée (en CFU/g de carpaccio). Ensuite un abaque a été utilisé où la concentration initiale de la suspension bactérienne utilisée pour tremper les carpaccios suffisait à déduire la charge du carpaccio inoculé volontairement.

Un protocole d'altération a été mis au point qui permettait un développement mesurable des différentes espèces : après inoculation par les compositions à tester, les échantillons ont été conditionnés sous-vide, puis placés 7 jours à 8°C, 24 heures à 22°C et enfin 6 jours à 8°C.

### 2) Mesures quantitatives des différents genres/groupes/espèces bactériennes

Les mesures quantitatives des différents genres/groupes/espèces bactériennes sont généralement effectuées par étalement sur milieux de cultures plus ou moins sélectifs. Un protocole pour établir ces mesures par PCR quantitative en temps réel (q-PCR) a été développé. Il a nécessité la mise au point (incluant le test de spécificité et d'efficacité) d'amorces qui permettent la mesure d'espèces/genres/groupes bactériens à partir de l'ADN bactérien extrait des carpaccios. Une fois l'efficacité et la spécificité des amorces testées, des gammes standard ont été réalisées pour avoir la correspondance Ct (donnée de q-PCR sur l'ADN extrait des carpaccios) / CFU (donnée expérimentale correspondant au nombre de bactéries inoculées sur les carpaccios standards) (par exemple, quantification de *Hafnia alvei* CIP57.31 par qPCR, voir figure 1).
Les amorces suivantes ont été utilisées :
- pour *Serratia*
   QSF01-HAL-F : TAACTTGGGAACTGCATTTGAAACTGGTC (SEQ ID NO :43)
   QSF01-HAL-R : CGCCACTGGTGTTCCTCCAGATC (SEQ ID NO :44)
- pour *Hafnia alvei*
   QSF02-HAL-F : ATTGGCAATTGGCGCTGAAGAAGGT (SEQ ID NO :45)
   QSF02-HAL-R : GCTCAGCCATATCAGCTGGGATC (SEQ ID NO :46)
- pour *Brochothrix thermosphacta*:
   QSF01-BTH-F : ACGAACGGATAAAGAGCTTGCTCTTTTG (SEQ ID NO :47)
   QSF01-BTH-R : CGAAACCGTCTTTCACTTGAACATCTTAT (SEQ ID NO :48)
   QSF03-BTH-F : GGACCAGAGGTTATCGAAACATTAACTG (SEQ ID NO :49)
   QSF03-BTH-R : TAATACCAGCAGCAGGAATTGCTT (SEQ ID NO :50)
- pour *Weisella viridescens*
   QSF01-WVI-F : TTGCTCAGATATGACGATGGACATTGCA (SEQ ID NO :51)
   QSF01-WVI-R : GACCATCTCTTAGTGATAGCAAGACCAT (SEQ ID NO :52)
- pour *Pseudomonas*
   QSF01-PSD-F : GGTCTTCGGATTGTAAAGCACTTTAAGTTG (SEQ ID NO :53)
   QSF01-PSD-R : GTAGTTAGCCGGTGCTTATTCTGTCG (SEQ ID NO :54)
- pour *Leuconostoc*
   QSF01-LCN2-F : GTGAAAGCCCGGAGCTCAACTC (SEQ ID NO :55)
   QSF01-LCN2-R : CTGGTGTTCTTCCACATATCTACGCATTC (SEQ ID NO :56)
- pour *Lactobacillus sakei*
   QMF01-LSA-F : CTGGCTATCCCGATACATACC (SEQ ID NO :57)
   QMF01-LSA-R : GCATATCTTGGCTACGGGCA (SEQ ID NO :58)
   QMF07-LSA-F : ATCTAGTAGACTCTGAATTTAAGGT (SEQ ID NO :59)
   QMF07-LSA-R : TTAACCGCTTCTAACACCTTTTGA (SEQ ID NO :60)
   QMF15-LSA-F : CTCTACCTCTGAAATGTCCAAG (SEQ ID NO :61)
   QMF15-LSA-R : TTTAGAGGTCTTCACCATTGTTG (SEQ ID NO :62)
   QMF16-LSA-F : ATTTATTCCAGCCAGCGTTTC (SEQ ID NO :63)
   QMF16-LSA-R : TGTGGAAAACATTCATGTACCG (SEQ ID NO :64)
   QMF21-LSA-F : AATTGGGTGCCTGCCGCG (SEQ ID NO :65)
   QMF21-LSA-R : GAATAAGAGCGCGAATACGG (SEQ ID NO :66)

### 3) Croissance des espèces altérantes du carpaccio

La figure 2 montre que le domaine expérimental est d'une grande variabilité car la croissance de chacune des espèces varie d'un lot de Carpaccio à un autre. La nature de l'écosystème microbien (qualitatif & quantitatif) naturellement présent sur l'aliment est l'un des facteurs majeurs qui influe sur cette variabilité. Sur un ensemble de plusieurs lots il est possible de dégager des tendances de croissances pour chacune des espèces : *e.g.* forte croissance de environ 3,5 ΔLog₁₀ CFU/g pour *Hafnia alvei* ou faible de environ 0,5 ΔLog₁₀ CFU/g pour *Weisseilla viridescens*. Cette notion de tendance globale sera également utilisée dans la mesure d'efficacité de la composition.

### 4) Mesure de l'effet barrière des compositions et de l'influence relative de chacune des souches au sein des compositions

On peut mesurer directement l'effet d'une composition par la mesure du ΔLog₁₀ CFU/g entre T_{14 jours} et T₀ pour les espèces altérantes.

Il apparait que la qualité de l'assemblage des souches est importante pour lutter contre l'altération et qu'il ne suffit pas d'utiliser une seule souche de *Lactobacillus sakei* ou d'assembler au hasard plusieurs souches pour obtenir un effet barrière.

### 5) Vérification de l'efficacité de la composition selon l'invention contre l'altération

La notion de composition la plus ou la moins efficace se définit par la valeur minimale ou maximale obtenue par le cumul de toutes les valeurs de Δlog10 CFU/g pour les bactéries altérantes. Cette mesure arbitraire permet d'estimer l'effet d'une composition sur l'ensemble des espèces d'altération. Ainsi, une composition active contre *Brochothrix thermosphacta* peut se révéler peu active contre les *Leuconostocs.*

Le tableau 1 ci-après montre l'efficacité globale de la composition selon l'invention par comparaison avec l'efficacité des 21 autres compositions de L. sakei et des témoins sans ajout de composition. Les résultats sont exprimés en ΔLog10 CFU/g pour les espèces cibles.

**Tableau 1**

| | *Enterobacteriaceae (Serratia*/*Hafnia)* | *Pseudomonadaceae (fragi*/*putida*/*flu orescens)* | *Leuconostocs (mesenteroides*/*carnosum)* | *Hafnia alvei* | *Brochothrix thermosphacta* |
|---|---|---|---|---|---|
| Contrôle non traité^{a} | 2,07 ± 1.16 | 2,18 ± 0.58 | 3,17 ± 0.83 | 4,01 ± 0.46 | 1,77 ± 0.66 |
| Composition de l'invention^{b} | 0.22 ± 0.68 | 0.13 ± 0.53 | 1.18 ± 0.46 | 2.91 ± 0.63 | 0.29 ± 0.47 |
| Autres compositions^{c} | 1.57 ± 0.99 | 0.43 ± 0.63 | 1.30 ± 0.32 | 3.98 ± 0.48 | 0.66 ± 0.92 |

| | | | | | |
|---|---|---|---|---|---|
| ^{a} Contrôle = 12 échantillons de Carpaccio non traités présentant une population naturelle de *L. sakei* ^{b} Composition de l'invention = 6 échantillons de Carpaccio traités avec la composition de la présente invention ^{c} Autres compositions = 21 échantillons de Carpaccio traités avec 21 cocktails différents de 4 souches de *L. sakei* | | | | | |

Le Tableau 1 montre, si l'on considère les valeurs médianes, que :
les 21 compositions testées présentaient des effets variables mais que la médiane des effets de ces 21 compositions diminuait par rapport à la moyenne observée en absence de toute composition ;
la composition selon l'invention est plus performante que les compositions les plus efficaces parmi les autres compositions testées. Ces résultats restent vrais si on considère les médianes par rapport à l'étape de criblage et par rapport aux témoins (sans composition). La composition selon la présente invention abolit quasiment la croissance de *Brochothrix thermosphacta*, limite très sérieusement celle des *Pseudomonas* et limite celle d'*Hafnia alvei* et des *Serratia.*

La figure 3 montre une nette diminution globale du CGE entre le témoin sans composition (médiane sur 6 lots de 13.8) et le témoin avec la composition selon la présente invention (médiane sur 6 lots de 4.3). Cette composition est donc plus efficace que la meilleure des autres compositions testées. Les résultats sur 3 lots des différentes combinaisons à 3 souches de la composition selon l'invention montrent que ces combinaisons sont en moyennes plus efficaces que toutes les autres combinaisons à 4 souches utilisées pendant l'étape de criblage. On observe également une plus grande amplitude de réponse avec certaines combinaisons à 3 souches qu'avec la composition à 4 souches. Enfin, la stabilité des effets sur le CGE semble plus robuste pour la composition à 4 souches que les combinaisons à 3 souches parmi les 4.

### 6) Identification de la composition selon l'invention

La composition selon l'invention possède une empreinte moléculaire unique, permettant de sélectionner des marqueurs moléculaires spécifiques à chacune des souches de la composition. Une quantification précise des souches de la composition selon la présente invention au sein d'un produit camé comme le Carpaccio est donc possible.

Le marqueur *katA* (SEQ ID NO :1), spécifique de l'espèce *L. sakei* (présent chez toutes les souches c'est à dire celles de la composition selon la présente invention mais aussi celles de la flore de *L. sakei* naturellement présente sur la viande) a été utilisé comme contrôle positif.

Les marqueurs de séquence SEQ ID NO :35, SEQ ID NO : 39, SEQ ID NO :21 et SEQ ID NO :26 sont chacun spécifique d'une seule des 4 souches composant la composition selon la présente invention.

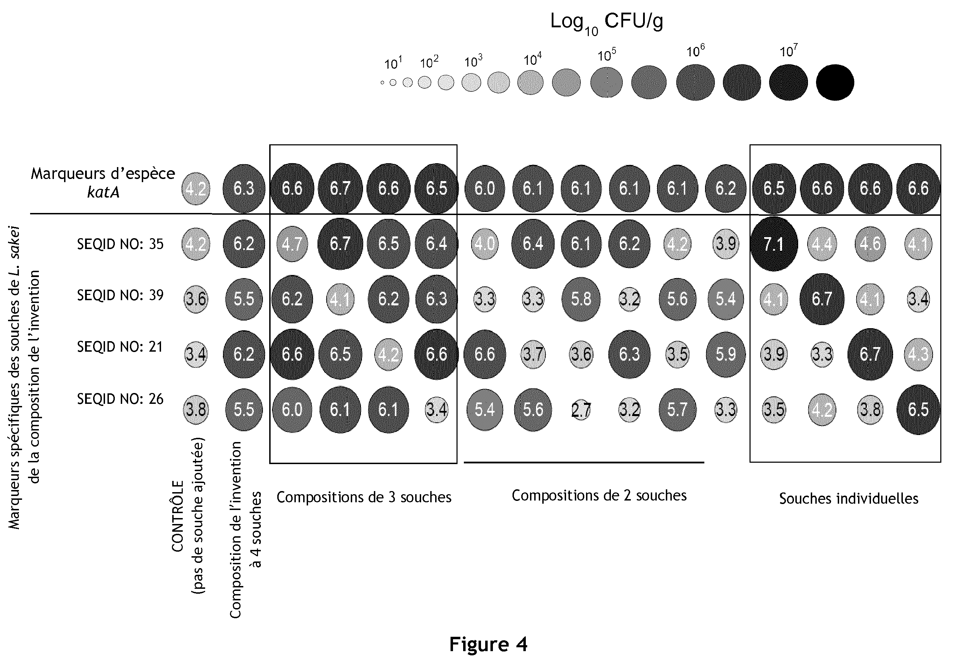

La figure 4 montre que :
- au 14^{e} jour (T14), sans ajout de composition (colonne de gauche) la population de *L. sakei* est de l'ordre de environ 10³-10⁴ CFU/g et est plus élevée jusqu'à des valeurs de l'ordre de environ 5.10⁵-10⁶ CFU/g dès lors que au moins une des souches de la composition a été inoculée ;
- les souches de *L. sakei* inoculées se développent, sans toutefois atteindre des niveaux susceptibles d'être altérants (la valeur max atteinte avec la composition de l'invention est de 6,3 Log₁₀, et de 6,6 Log₁₀ à 7.1 Log₁₀ CFU/g avec des mélanges à 1, 2 ou 3 souches, valeurs qui ne sont pas considérées comme altérantes) ;
- l'utilisation du marqueur *katA* permet de déceler une population globale de *L. sakei* plus importante dès lors qu'au moins une souche de la composition est ajoutée (environ 6 Log₁₀ CFU/g avec ajout de souches contre environ 4 Log₁₀ CFU/g sans ajout).
- les souches se sont développées chacune de manière similaire que ce soit dans la composition selon l'invention, pour les souches seules ou dans les combinaisons de trois ou deux souches parmi les quatre de la composition.
- à T₁₄ⱼₒᵤᵣₛ chaque souche de la composition est bien quantifiable grâce à son marqueur spécifique (dans les combinaisons à une, deux ou trois souches il est facile de repérer chacune des souches qui a été inoculée (environ 5,5-7 Log₁₀ CFU/g contre environ 3 à 4 Log₁₀ CFU/g de bruit de fond correspondant à la population endogène de *L. sakei*).
- la composition est donc de fait identifiable et quantifiable.

## Revendications

1. Composition comprenant au moins trois souches différentes de bactéries *L. sakei,* dans laquelle :
- ladite première souche de bactéries *L. sakei* comprend dans son génome la séquence suivante :
• SEQ ID NO: 35 ou une séquence présentant au moins 95% d'identité avec celle-ci,
- ladite deuxième souche de bactéries *L. sakei* comprend dans son génome la séquence suivante :
• SEQ ID NO: 21 ou une séquence présentant au moins 95% d'identité avec celle-ci,
- ladite troisième souche de bactéries *L. sakei* comprend dans son génome la séquence suivante :
• SEQ ID NO: 26 ou une séquence présentant au moins 95% d'identité avec celle-ci.

2. Composition selon la revendication 1, dans laquelle ladite première souche de bactéries *L. sakei* comprend dans son génome les séquences suivantes :
• SEQ ID NO: 42, et
• SEQ ID NO: 35 ou une séquence présentant au moins 95% d'identité avec celle-ci,
de préférence ladite première souche de bactéries *L. sakei* comprend dans son génome les séquences suivantes :
• SEQ ID NO: 2 ou une séquence présentant au moins 95% d'identité avec celle-ci,
• SEQ ID NO: 34 ou une séquence présentant au moins 95% d'identité avec celle-ci,
• SEQ ID NO: 42, et
• SEQ ID NO: 35 ou une séquence présentant au moins 95% d'identité avec celle-ci;
de préférence ladite première souche de bactéries *L. sakei* comprend dans son génome les séquences suivantes :
• SEQ ID NO: 2 ou une séquence présentant au moins 95% d'identité avec celle-ci,
• SEQ ID NO: 4 ou une séquence présentant au moins 95% d'identité avec celle-ci,
• SEQ ID NO: 6 ou une séquence présentant au moins 95% d'identité avec celle-ci,
• SEQ ID NO: 7 ou une séquence présentant au moins 95% d'identité avec celle-ci,
• SEQ ID NO: 8 ou une séquence présentant au moins 95% d'identité avec celle-ci,
• SEQ ID NO: 9 ou une séquence présentant au moins 95% d'identité avec celle-ci,
• SEQ ID NO: 10 ou une séquence présentant au moins 95% d'identité avec celle-ci,
• SEQ ID NO: 13 ou une séquence présentant au moins 95% d'identité avec celle-ci,
• SEQ ID NO: 15 ou une séquence présentant au moins 95% d'identité avec celle-ci,
• SEQ ID NO: 19 ou une séquence présentant au moins 95% d'identité avec celle-ci,
• SEQ ID NO: 32 ou une séquence présentant au moins 95% d'identité avec celle-ci,
• SEQ ID NO: 34 ou une séquence présentant au moins 95% d'identité avec celle-ci,
• SEQ ID NO: 42,
• SEQ ID NO: 35 ou une séquence présentant au moins 95% d'identité avec celle-ci,
• SEQ ID NO: 36 ou une séquence présentant au moins 95% d'identité avec celle-ci, et
• SEQ ID NO: 38 ou une séquence présentant au moins 95% d'identité avec celle-ci.

3. Composition selon la revendication 1 ou 2, dans laquelle ladite deuxième souche de bactéries *L. sakei* comprend dans son génome les séquences suivantes :
• SEQ ID NO: 21 ou une séquence présentant au moins 95% d'identité avec celle-ci, et
• SEQ ID NO: 40 ou une séquence présentant au moins 95% d'identité avec celle-ci;
de préférence, ladite deuxième souche de bactéries *L. sakei* comprend dans son génome les séquences suivantes :
• SEQ ID NO: 16 ou une séquence présentant au moins 95% d'identité avec celle-ci,
• SEQ ID NO: 17 ou une séquence présentant au moins 95% d'identité avec celle-ci,
• SEQ ID NO: 21 ou une séquence présentant au moins 95% d'identité avec celle-ci, et
• SEQ ID NO: 40 ou une séquence présentant au moins 95% d'identité avec celle-ci;
de préférence ladite deuxième souche de bactéries *L. sakei* comprend dans son génome les séquences suivantes :
• SEQ ID NO: 4 ou une séquence présentant au moins 95% d'identité avec celle-ci,
• SEQ ID NO: 8 ou une séquence présentant au moins 95% d'identité avec celle-ci,
• SEQ ID NO: 9 ou une séquence présentant au moins 95% d'identité avec celle-ci,
• SEQ ID NO: 11 ou une séquence présentant au moins 95% d'identité avec celle-ci,
• SEQ ID NO: 12 ou une séquence présentant au moins 95% d'identité avec celle-ci,
• SEQ ID NO: 16 ou une séquence présentant au moins 95% d'identité avec celle-ci,
• SEQ ID NO: 17 ou une séquence présentant au moins 95% d'identité avec celle-ci,
• SEQ ID NO: 20 ou une séquence présentant au moins 95% d'identité avec celle-ci,
• SEQ ID NO: 21 ou une séquence présentant au moins 95% d'identité avec celle-ci,
• SEQ ID NO: 36 ou une séquence présentant au moins 95% d'identité avec celle-ci,
• SEQ ID NO: 38 ou une séquence présentant au moins 95% d'identité avec celle-ci,
• SEQ ID NO: 40 ou une séquence présentant au moins 95% d'identité avec celle-ci,
de préférence ladite souche comprend en outre une, de préférence, 2, 3, 4, 5, 6, 7, 8, 9, 10, 11, 12, 13, et encore préférentiellement 7, 8, 9, 10, 11, 12 des séquences choisies dans le groupe constitué par les séquences: SEQ ID NO: 5, SEQ ID NO: 10, SEQ ID NO: 14, SEQ ID NO: 15, SEQ ID NO: 18, SEQ ID NO: 19, SEQ ID NO: 24, SEQ ID NO: 29, SEQ ID NO: 31, SEQ ID NO: 32, SEQ ID NO: 33, SEQ ID NO: 37, et SEQ ID NO: 41 ou une séquence présentant au moins 95% d'identité avec celles-ci respectivement.

4. Composition selon l'une quelconque des revendications 1 à 3, dans laquelle ladite troisième souche de bactéries *L. sakei* comprend dans son génome les séquences suivantes :
• SEQ ID NO: 23 ou une séquence présentant au moins 95% d'identité avec celle-ci, et
• SEQ ID NO: 26 ou une séquence présentant au moins 95% d'identité avec celle-ci,
de préférence ladite troisième souche de bactéries *L. sakei* comprend dans son génome au moins les séquences suivantes :
• SEQ ID NO :22 ou une séquence présentant au moins 95% d'identité avec celle-ci,
• SEQ ID NO: 23 ou une séquence présentant au moins 95% d'identité avec celle-ci, et
• SEQ ID NO: 26 ou une séquence présentant au moins 95% d'identité avec celle-ci,
de préférence ladite troisième souche de bactéries *L. sakei* comprend dans son génome au moins les séquences suivantes :
• SEQ ID NO: 7 ou une séquence présentant au moins 95% d'identité avec celle-ci,
• SEQ ID NO: 13 ou une séquence présentant au moins 95% d'identité avec celle-ci,
• SEQ ID NO: 15 ou une séquence présentant au moins 95% d'identité avec celle-ci,
• SEQ ID NO: 20 ou une séquence présentant au moins 95% d'identité avec celle-ci,
• SEQ ID NO: 22 ou une séquence présentant au moins 95% d'identité avec celle-ci,
• SEQ ID NO: 23 ou une séquence présentant au moins 95% d'identité avec celle-ci, SEQ ID NO: 26 ou une séquence présentant au moins 95% d'identité avec celle-ci,
et de préférence ladite souche comprend en outre une, de préférence, 2, 3, 4, 5, 6, 7, 8, 9, 10, 11, 12, 13, 14 ou 15 et encore préférentiellement 5, 6, 7, 8, 9 des séquences choisies dans le groupe constitué par les séquences: SEQ ID NO: 3, SEQ ID NO: 5, SEQ ID NO: 9, SEQ ID NO: 12, SEQ ID NO: 14, SEQ ID NO: 25, SEQ ID NO: 27, SEQ ID NO: 28, SEQ ID NO: 29, SEQ ID NO: 30, SEQ ID NO: 31, SEQ ID NO: 36, SEQ ID NO: 37, SEQ ID NO: 38, et SEQ ID NO: 41 ou une séquence présentant au moins 95% d'identité avec celles-ci respectivement.

5. Composition selon l'une quelconque des revendications 1 à 4, comprenant en outre au moins une quatrième souche de *L. sakei* différente desdites première, deuxième et troisième souches telles que définies selon l'une quelconque des revendications 1 à 4.

6. Composition selon la revendication 5, dans laquelle ladite quatrième souche de bactéries *L. sakei* comprend dans son génome au moins la séquence SEQ ID NO : 39 ou une séquence présentant au moins 95% d'identité avec celle-ci.

7. Composition selon l'une quelconque des revendications 5 ou 6, dans laquelle ladite quatrième souche comprend dans son génome au moins les séquences suivantes :
• SEQ ID NO: 4 ou une séquence présentant au moins 95% d'identité avec celle-ci,
• SEQ ID NO: 6 ou une séquence présentant au moins 95% d'identité avec celle-ci,
• SEQ ID NO: 7 ou une séquence présentant au moins 95% d'identité avec celle-ci,
• SEQ ID NO: 8 ou une séquence présentant au moins 95% d'identité avec celle-ci,
• SEQ ID NO: 9 ou une séquence présentant au moins 95% d'identité avec celle-ci,
• SEQ ID NO: 15 ou une séquence présentant au moins 95% d'identité avec celle-ci,
• SEQ ID NO: 31 ou une séquence présentant au moins 95% d'identité avec celle-ci,
• SEQ ID NO: 36 ou une séquence présentant au moins 95% d'identité avec celle-ci,
• SEQ ID NO: 39 ou une séquence présentant au moins 95% d'identité avec celle-ci,
et de préférence ladite souche comprend en outre une, de préférence, 1, 2, 3, 4, 5 et encore préférentiellement 1 ou 2 des séquences choisies dans le groupe constitué par les séquences: SEQ ID NO: 10, SEQ ID NO: 12, SEQ ID NO: 19, SEQ ID NO: 32 et SEQ ID NO: 38 ou une séquence présentant au moins 95% d'identité avec celles-ci respectivement.

8. Composition selon l'une quelconque des revendications précédentes, comprenant au moins les 4 souches de *L. sakei* déposées auprès de la CNCM sous les numéros suivants : CNCM 1-4393, CNCM 1-4394, CNCM 1-4395 et CNCM 1-4396.

9. Utilisation d'une composition telle que définie dans l'une quelconque des revendications 1 à 8, pour la conservation d'un produit comestible, de préférence d'un produit carné, en particulier de la viande de boeuf et encore plus particulièrement du carpaccio de boeuf ou du boeuf haché et de manière particulièrement préférée du carpaccio de boeuf.

10. Méthode de conservation d'un produit comestible comprenant une étape de mise en contact dudit produit comestible avec une composition telle que définie selon l'une quelconque des revendications 1 à 8.

11. Produit comestible comprenant une composition telle que définie selon l'une quelconque des revendications 1 à 8.

## Patentansprüche

1. Zusammensetzung, umfassend mindestens drei verschiedene Bakterienstämme *L. sakei,* wobei:
- der erste Bakterienstamm *L. sakei* in seinem Genom die folgende Sequenz umfasst:
- SEQ ID NO: 35 oder eine Sequenz, die mindestens 95 % Identität mit dieser aufweist,
- der zweite Bakterienstamm *L. sakei* in seinem Genom die folgende Sequenz umfasst:
- SEQ ID NO: 21 oder eine Sequenz, die mindestens 95 % Identität mit dieser aufweist,
- der dritte Bakterienstamm *L. sakei* in seinem Genom die folgende Sequenz umfasst:
- SEQ ID NO: 26 oder eine Sequenz, die mindestens 95 % Identität mit dieser aufweist.

2. Zusammensetzung nach Anspruch 1, wobei der erste Bakterienstamm *L. sakei* in seinem Genom die folgenden Sequenzen umfasst:
- SEQ ID NO: 42, und
- SEQ ID NO: 35 oder eine Sequenz, die mindestens 95 % Identität mit dieser aufweist,
vorzugsweise der erste Bakterienstamm *L. sakei* in seinem Genom die folgenden Sequenzen umfasst:
- SEQ ID NO: 2 oder eine Sequenz, die mindestens 95 % Identität mit dieser aufweist,
- SEQ ID NO: 34 oder eine Sequenz, die mindestens 95 % Identität mit dieser aufweist,
- SEQ ID NO: 42, und
- SEQ ID NO: 35 oder eine Sequenz, die mindestens 95 % Identität mit dieser aufweist,
vorzugsweise der erste Bakterienstamm *L. sakei* in seinem Genom die folgenden Sequenzen umfasst:
- SEQ ID NO: 2 oder eine Sequenz, die mindestens 95 % Identität mit dieser aufweist,
- SEQ ID NO: 4 oder eine Sequenz, die mindestens 95 % Identität mit dieser aufweist,
- SEQ ID NO: 6 oder eine Sequenz, die mindestens 95 % Identität mit dieser aufweist,
- SEQ ID NO: 7 oder eine Sequenz, die mindestens 95 % Identität mit dieser aufweist,
- SEQ ID NO: 8 oder eine Sequenz, die mindestens 95 % Identität mit dieser aufweist,
- SEQ ID NO: 9 oder eine Sequenz, die mindestens 95 % Identität mit dieser aufweist,
- SEQ ID NO: 10 oder eine Sequenz, die mindestens 95 % Identität mit dieser aufweist,
- SEQ ID NO: 13 oder eine Sequenz, die mindestens 95 % Identität mit dieser aufweist,
- SEQ ID NO: 15 oder eine Sequenz, die mindestens 95 % Identität mit dieser aufweist,
- SEQ ID NO: 19 oder eine Sequenz, die mindestens 95 % Identität mit dieser aufweist,
- SEQ ID NO: 32 oder eine Sequenz, die mindestens 95 % Identität mit dieser aufweist,
- SEQ ID NO: 34 oder eine Sequenz, die mindestens 95 % Identität mit dieser aufweist,
- SEQ ID NO: 42,
- SEQ ID NO: 35 oder eine Sequenz, die mindestens 95 % Identität mit dieser aufweist,
- SEQ ID NO: 36 oder eine Sequenz, die mindestens 95 % Identität mit dieser aufweist, und
- SEQ ID NO: 38 oder eine Sequenz, die mindestens 95 % Identität mit dieser aufweist.

3. Zusammensetzung nach Anspruch 1 oder 2, wobei der zweite Bakterienstamm *L. sakei* in seinem Genom die folgenden Sequenzen umfasst:
- SEQ ID NO: 21 oder eine Sequenz, die mindestens 95 % Identität mit dieser aufweist, und
- SEQ ID NO: 40 oder eine Sequenz, die mindestens 95 % Identität mit dieser aufweist,
vorzugsweise der zweite Bakterienstamm *L. sakei* in seinem Genom die folgenden Sequenzen umfasst:
- SEQ ID NO: 16 oder eine Sequenz, die mindestens 95 % Identität mit dieser aufweist,
- SEQ ID NO: 17 oder eine Sequenz, die mindestens 95 % Identität mit dieser aufweist,
- SEQ ID NO: 21 oder eine Sequenz, die mindestens 95 % Identität mit dieser aufweist, und
- SEQ ID NO: 40 oder eine Sequenz, die mindestens 95 % Identität mit dieser aufweist,
vorzugsweise der zweite Bakterienstamm *L. sakei* in seinem Genom die folgenden Sequenzen umfasst:
- SEQ ID NO: 4 oder eine Sequenz, die mindestens 95 % Identität mit dieser aufweist,
- SEQ ID NO: 8 oder eine Sequenz, die mindestens 95 % Identität mit dieser aufweist,
- SEQ ID NO: 9 oder eine Sequenz, die mindestens 95 % Identität mit dieser aufweist,
- SEQ ID NO: 11 oder eine Sequenz, die mindestens 95 % Identität mit dieser aufweist,
- SEQ ID NO: 12 oder eine Sequenz, die mindestens 95 % Identität mit dieser aufweist,
- SEQ ID NO: 16 oder eine Sequenz, die mindestens 95 % Identität mit dieser aufweist,
- SEQ ID NO: 17 oder eine Sequenz, die mindestens 95 % Identität mit dieser aufweist,
- SEQ ID NO: 20 oder eine Sequenz, die mindestens 95 % Identität mit dieser aufweist,
- SEQ ID NO: 21 oder eine Sequenz, die mindestens 95 % Identität mit dieser aufweist,
- SEQ ID NO: 36 oder eine Sequenz, die mindestens 95 % Identität mit dieser aufweist,
- SEQ ID NO: 38 oder eine Sequenz, die mindestens 95 % Identität mit dieser aufweist,
- SEQ ID NO: 40 oder eine Sequenz, die mindestens 95 % Identität mit dieser aufweist,
vorzugsweise der Stamm außerdem eine, insbesondere 2, 3, 4, 5, 6, 7, 8, 9, 10, 11, 12, 13 und noch besser 7, 8, 9, 10, 11, 12 der Sequenzen umfasst, ausgewählt aus der Gruppe, bestehend aus den Sequenzen:
SEQ ID NO: 5, SEQ ID NO: 10, SEQ ID NO: 14, SEQ ID NO: 15, SEQ ID NO: 18, SEQ ID NO: 19, SEQ ID NO: 24, SEQ ID NO: 29, SEQ ID NO: 31, SEQ ID NO: 32, SEQ ID NO: 33, SEQ ID NO: 37 und SEQ ID NO: 41 oder eine Sequenz, die jeweils mindestens 95 % Identität mit diesen aufweist.

4. Zusammensetzung nach einem der Ansprüche 1 bis 3, wobei der dritte Bakterienstamm *L. sakei* in seinem Genom die folgenden Sequenzen umfasst:
- SEQ ID NO: 23 oder eine Sequenz, die mindestens 95 % Identität mit dieser aufweist, und
- SEQ ID NO: 26 oder eine Sequenz, die mindestens 95 % Identität mit dieser aufweist,
vorzugsweise der dritte Bakterienstamm *L. sakei* in seinem Genom mindestens die folgenden Sequenzen umfasst:
- SEQ ID NO: 22 oder eine Sequenz, die mindestens 95 % Identität mit dieser aufweist,
- SEQ ID NO: 23 oder eine Sequenz, die mindestens 95 % Identität mit dieser aufweist, und
- SEQ ID NO: 26 oder eine Sequenz, die mindestens 95 % Identität mit dieser aufweist,
vorzugsweise der dritte Bakterienstamm *L*. *sakei* in seinem Genom mindestens die folgenden Sequenzen umfasst:
- SEQ ID NO: 7 oder eine Sequenz, die mindestens 95 % Identität mit dieser aufweist,
- SEQ ID NO: 13 oder eine Sequenz, die mindestens 95 % Identität mit dieser aufweist,
- SEQ ID NO: 15 oder eine Sequenz, die mindestens 95 % Identität mit dieser aufweist,
- SEQ ID NO: 20 oder eine Sequenz, die mindestens 95 % Identität mit dieser aufweist,
- SEQ ID NO: 22 oder eine Sequenz, die mindestens 95 % Identität mit dieser aufweist,
- SEQ ID NO: 23 oder eine Sequenz, die mindestens 95 % Identität mit dieser aufweist, und SEQ ID NO: 26 oder eine Sequenz, die mindestens 95 % Identität mit dieser aufweist,
und vorzugsweise der Stamm außerdem eine, insbesondere 2, 3, 4, 5, 6, 7, 8, 9, 10, 11, 12, 13, 14 oder 15 und noch besser 5, 6, 7, 8, 9 der Sequenzen umfasst, ausgewählt aus der Gruppe, bestehend aus den Sequenzen SEQ ID NO: 3, SEQ ID NO: 5, SEQ ID NO: 9, SEQ ID NO: 12, SEQ ID NO: 14, SEQ ID NO: 25, SEQ ID NO: 27, SEQ ID NO: 28, SEQ ID NO: 29, SEQ ID NO: 30, SEQ ID NO: 31, SEQ ID NO: 36, SEQ ID NO: 37, SEQ ID NO: 38 und SEQ ID NO: 41 oder eine Sequenz, die jeweils mindestens 95 % Identität mit diesen aufweist.

5. Zusammensetzung nach einem der Ansprüche 1 bis 4, umfassend außerdem mindestens einen vierten Stamm von *L. sakei,* der verschieden vom ersten, zweiten und dritten Stamm ist, wie nach einem der Ansprüche 1 bis 4 definiert.

6. Zusammensetzung nach Anspruch 5, wobei der vierte Bakterienstamm *L. sakei* in seinem Genom mindestens die Sequenz SEQ ID NO: 39 oder eine Sequenz umfasst, die mindestens 95 % Identität mit dieser aufweist.

7. Zusammensetzung nach einem der Ansprüche 5 oder 6, wobei der vierte Stamm in seinem Genom mindestens die folgenden Sequenzen umfasst:
- SEQ ID NO: 4 oder eine Sequenz, die mindestens 95 % Identität mit dieser aufweist,
- SEQ ID NO: 6 oder eine Sequenz, die mindestens 95 % Identität mit dieser aufweist,
- SEQ ID NO: 7 oder eine Sequenz, die mindestens 95 % Identität mit dieser aufweist,
- SEQ ID NO: 8 oder eine Sequenz, die mindestens 95 % Identität mit dieser aufweist,
- SEQ ID NO: 9 oder eine Sequenz, die mindestens 95 % Identität mit dieser aufweist,
- SEQ ID NO: 15 oder eine Sequenz, die mindestens 95 % Identität mit dieser aufweist,
- SEQ ID NO: 31 oder eine Sequenz, die mindestens 95 % Identität mit dieser aufweist,
- SEQ ID NO: 36 oder eine Sequenz, die mindestens 95 % Identität mit dieser aufweist,
- SEQ ID NO: 39 oder eine Sequenz, die mindestens 95 % Identität mit dieser aufweist,
und vorzugsweise der Stamm außerdem eine, insbesondere 1, 2, 3, 4, 5 und noch besser 1 oder 2 der Sequenzen umfasst, ausgewählt aus der Gruppe, bestehend aus den Sequenzen SEQ ID NO: 10, SEQ ID NO: 12, SEQ ID NO: 19, SEQ ID NO: 32 und SEQ ID NO: 38 oder eine Sequenz, die jeweils mindestens 95 % Identität mit diesen aufweist.

8. Zusammensetzung nach einem der vorhergehenden Ansprüche, umfassend mindestens die 4 Stämme von *L*. *sakei,* die in der CNCM unter den folgenden Nummern hinterlegt sind: CNCM I-4393, CNCM I-4394, CNCM I-4395 und CNCM I-4396.

9. Verwendung einer Zusammensetzung, wie in einem der Ansprüche 1 bis 8 definiert, für die Konservierung eines Nahrungsmittels, vorzugsweise eines Fleischprodukts, besonders von Rindfleisch und insbesondere von Rindfleisch-Carpaccio oder von gehacktem Rindfleisch und besonders bevorzugt von Rindfleisch-Carpaccio.

10. Verfahren zur Konservierung eines Nahrungsmittels, umfassend einen Schritt des In-Kontakt-Bringens des Nahrungsmittels mit einer Zusammensetzung, wie nach einem der Ansprüche 1 bis 8 definiert.

11. Nahrungsmittel, umfassend eine Zusammensetzung, wie nach einem der Ansprüche 1 bis 8 definiert.

## Claims

1. A composition comprising at least three different strains of *L. sakei* bacteria, wherein:
- said first strain of *L. sakei* bacteria comprises in its genome the following sequence:
• SEQ ID NO: 35 or a sequence exhibiting at least 95 % identity thereto,
- said second strain of *L. sakei* bacteria comprises in its genome the following sequence:
• SEQ ID NO: 21 or a sequence exhibiting at least 95 % identity thereto,
- said third strain of *L. sakei* bacteria comprises in its genome the following sequence:
• SEQ ID NO: 26 or a sequence exhibiting at least 95 % identity thereto.

2. The composition according to claim 1, wherein said first strain of *L. sakei* bacteria comprises in its genome the following sequences:
• SEQ ID NO: 42, and
• SEQ ID NO: 35 or a sequence exhibiting at least 95 % identity thereto,
preferably said first strain of *L. sakei* bacteria comprises in its genome the following sequences:
• SEQ ID NO: 2 or a sequence exhibiting at least 95 % identity thereto,
• SEQ ID NO: 34 or a sequence exhibiting at least 95 % identity thereto,
• SEQ ID NO: 42, and
• SEQ ID NO: 35 or a sequence exhibiting at least 95 % identity thereto;
preferably said first strain of *L. sakei* bacteria comprises in its genome the following sequences:
• SEQ ID NO: 2 or a sequence exhibiting at least 95 % identity thereto,
• SEQ ID NO: 4 or a sequence exhibiting at least 95 % identity thereto,
• SEQ ID NO: 6 or a sequence exhibiting at least 95 % identity thereto,
• SEQ ID NO: 7 or a sequence exhibiting at least 95 % identity thereto,
• SEQ ID NO: 8 or a sequence exhibiting at least 95 % identity thereto,
• SEQ ID NO: 9 or a sequence exhibiting at least 95 % identity thereto,
• SEQ ID NO: 10 or a sequence exhibiting at least 95 % identity thereto,
• SEQ ID NO: 13 or a sequence exhibiting at least 95 % identity thereto,
• SEQ ID NO: 15 or a sequence exhibiting at least 95 % identity thereto,
• SEQ ID NO: 19 or a sequence exhibiting at least 95 % identity thereto,
• SEQ ID NO: 32 or a sequence exhibiting at least 95 % identity thereto,
• SEQ ID NO: 34 or a sequence exhibiting at least 95 % identity thereto,
• SEQ ID NO: 42,
• SEQ ID NO: 35 or a sequence exhibiting at least 95 % identity thereto,
• SEQ ID NO: 36 or a sequence exhibiting at least 95 % identity thereto, and
• SEQ ID NO: 38 or a sequence exhibiting at least 95 % identity thereto.

3. The composition according to claim 1 or 2, wherein said second strain of *L. sakei* bacteria comprises in its genome the following sequences:
• SEQ ID NO: 21 or a sequence exhibiting at least 95 % identity thereto, and
• SEQ ID NO: 40 or a sequence exhibiting at least 95 % identity thereto;
preferably said second strain of *L. sakei* bacteria comprises in its genome the following sequences:
• SEQ ID NO: 16 or a sequence exhibiting at least 95 % identity thereto,
• SEQ ID NO: 17 or a sequence exhibiting at least 95 % identity thereto,
• SEQ ID NO: 21 or a sequence exhibiting at least 95 % identity thereto, and
• SEQ ID NO: 40 or a sequence exhibiting at least 95 % identity thereto;
preferably said second strain of *L. sakei* bacteria comprises in its genome the following sequences:
• SEQ ID NO: 4 or a sequence exhibiting at least 95 % identity thereto,
• SEQ ID NO: 8 or a sequence exhibiting at least 95 % identity thereto,
• SEQ ID NO: 9 or a sequence exhibiting at least 95 % identity thereto,
• SEQ ID NO: 11 or a sequence exhibiting at least 95 % identity thereto,
• SEQ ID NO: 12 or a sequence exhibiting at least 95 % identity thereto,
• SEQ ID NO: 16 or a sequence exhibiting at least 95 % identity thereto,
• SEQ ID NO: 17 or a sequence exhibiting at least 95 % identity thereto,
• SEQ ID NO: 20 or a sequence exhibiting at least 95 % identity thereto,
• SEQ ID NO: 21 or a sequence exhibiting at least 95 % identity thereto,
• SEQ ID NO: 36 or a sequence exhibiting at least 95 % identity thereto,
• SEQ ID NO: 38 or a sequence exhibiting at least 95 % identity thereto,
• SEQ ID NO: 40 or a sequence exhibiting at least 95 % identity thereto,
preferably said strain further comprises one, preferably, 2, 3, 4, 5, 6, 7, 8, 9, 10, 11, 12, 13, and more preferably 7, 8, 9, 10, 11, 12 of the sequences selected from the group consisting of the sequences: SEQ ID NO: 5, SEQ ID NO: 10, SEQ ID NO: 14, SEQ ID NO: 15, SEQ ID NO: 18, SEQ ID NO: 19, SEQ ID NO: 24, SEQ ID NO: 29, SEQ ID NO: 31, SEQ ID NO: 32, SEQ ID NO: 33, SEQ ID NO: 37, and SEQ ID NO: 41 or a sequence exhibiting at least 95 % identity thereto respectively.

4. The composition according to any of claims 1 to 3, wherein said third strain of *L. sakei* bacteria comprises in its genome the following sequences:
• SEQ ID NO: 23 or a sequence exhibiting at least 95 % identity thereto, and
• SEQ ID NO: 26 or a sequence exhibiting at least 95 % identity thereto,
preferably said third strain of *L. sakei* bacteria comprises in its genome at least the following sequences:
• SEQ ID NO: 22 or a sequence exhibiting at least 95 % identity thereto,
• SEQ ID NO: 23 or a sequence exhibiting at least 95 % identity thereto, and
• SEQ ID NO: 26 or a sequence exhibiting at least 95 % identity thereto,
preferably said third strain of *L. sakei* bacteria comprises in its genome at least the following sequences:
• SEQ ID NO: 7 or a sequence exhibiting at least 95 % identity thereto,
• SEQ ID NO: 13 or a sequence exhibiting at least 95 % identity thereto,
• SEQ ID NO: 15 or a sequence exhibiting at least 95 % identity thereto,
• SEQ ID NO: 20 or a sequence exhibiting at least 95 % identity thereto,
• SEQ ID NO: 22 or a sequence exhibiting at least 95 % identity thereto,
• SEQ ID NO: 23 or a sequence exhibiting at least 95 % identity thereto, SEQ ID NO: 26 or a sequence exhibiting at least 95 % identity thereto,
and preferably said strain further comprises one, preferably 2, 3, 4, 5, 6, 7, 8, 9, 10, 11, 12, 13, 14, or 15, and more preferably 5, 6, 7, 8, 9 of the sequences selected from the group consisting of the sequences: SEQ ID NO: 3, SEQ ID NO: 5, SEQ ID NO: 9, SEQ ID NO: 12, SEQ ID NO: 14, SEQ ID NO: 25, SEQ ID NO: 27, SEQ ID NO: 28, SEQ ID NO: 29, SEQ ID NO: 30, SEQ ID NO: 31, SEQ ID NO: 36, SEQ ID NO: 37, SEQ ID NO: 38, and SEQ ID NO: 41 or a sequence exhibiting at least 95 % identity thereto respectively.

5. The composition according to any of claims 1 to 4, further comprising at least a fourth strain of *L. sakei* different from said first, second and third strains as defined according to any of claims 1 to 4.

6. The composition according to claim 5, wherein said fourth strain of *L. sakei* bacteria comprises in its genome at least the sequence SEQ ID NO: 39 or a sequence exhibiting at least 95 % identity thereto.

7. The composition according to any of claims 5 or 6, wherein said fourth strain comprises in its genome at least the following sequences:
• SEQ ID NO: 4 or a sequence exhibiting at least 95 % identity thereto,
• SEQ ID NO: 6 or a sequence exhibiting at least 95 % identity thereto,
• SEQ ID NO: 7 or a sequence exhibiting at least 95 % identity thereto,
• SEQ ID NO: 8 or a sequence exhibiting at least 95 % identity thereto,
• SEQ ID NO: 9 or a sequence exhibiting at least 95 % identity thereto,
• SEQ ID NO: 15 or a sequence exhibiting at least 95 % identity thereto,
• SEQ ID NO: 31 or a sequence exhibiting at least 95 % identity thereto,
• SEQ ID NO: 36 or a sequence exhibiting at least 95 % identity thereto,
• SEQ ID NO: 39 or a sequence exhibiting at least 95 % identity thereto,
and preferably said strain further comprises one, preferably, 1, 2, 3, 4, 5, and even preferably 1 or 2 of the sequences selected from the group consisting of the sequences: SEQ ID NO: 10, SEQ ID NO: 12, SEQ ID NO: 19, SEQ ID NO: 32 and SEQ ID NO: 38 or a sequence exhibiting at least 95 % identity thereto respectively.

8. The composition according to any of the preceding claims, comprising at least the 4 strains of *L. sakei* deposited with the CNCM as the following numbers: CNCM I-4393, CNCM I-4394, CNCM I-4395, and CNCM I-4396.

9. A use of a composition as defined in any of claims 1 to 8, for preserving an edible product, preferably a meat product, in particular beef meat and even more particularly beef carpaccio or chopped beef and particularly preferably beef carpaccio.

10. A method for preserving an edible product comprising a step of contacting said edible product with a composition as defined according to any of claims 1 to 8.

11. An edible product comprising a composition as defined according to any of claims 1 to 8.
